(19) Europäisches Patentamt
European Patent Office
Office européen des brevets

(11) Numéro de publication : **0 462 892 A1**

# (12) DEMANDE DE BREVET EUROPEEN

(21) Numéro de dépôt : **91401643.1**

(22) Date de dépôt : **18.06.91**

(51) Int. Cl.$^5$ : **C12N 15/52**, C12P 19/42, C12N 1/21, // (C12N15/52, C12R1:42), (C12N1/21, C12R1:19)

(30) Priorité : **20.06.90 FR 9007724**

(43) Date de publication de la demande : **27.12.91 Bulletin 91/52**

(84) Etats contractants désignés : **AT BE CH DE DK ES FR GB GR IT LI LU NL SE**

(71) Demandeur : **Rambach, Alain**
**73 Bld du Montparnasse**
**F-75006 Paris (FR)**

(72) Inventeur : **Rambach, Alain**
**73 Bld du Montparnasse**
**F-75006 Paris (FR)**

(74) Mandataire : **Warcoin, Jacques et al**
**Cabinet Régimbeau 26, avenue Kléber**
**F-75116 Paris (FR)**

(54) **Souches de E.Coli productrices de vitamine B12 et procédé de préparation de la vitamine B12 par culture de ces souches.**

(57) L'invention concerne des souches de E.Coli productrices de vitamine B12 transformées par un ADN hétérologue comportant tout ou partie de la région cobI et permettant l'expression de ladite région dans E.Coli. En particulier, la région cobI est la région de Salmonella typhimurium.

EP 0 462 892 A1

L'invention a pour objet de nouvelles souches productrices de vitamine B12 ainsi qu'un procédé de préparation de vitamine B12 par culture desdites souches.

La vitamine B12 encore appelée cobalamine est essentielle à l'homme et aux animaux non ruminants.

Ils la trouvent essentiellement dans une alimentation carnée. C'est pourquoi, dans les élevages industriels d'animaux dont l'alimentation est uniquement à base de protéines végétales, il faut, pour assurer à ces animaux une croissance normale, incorporer la vitamine B12 à leur nourriture.

La vitamine B12 est actuellement essentiellement préparée par fermentation bactérienne et de nombreux brevets décrivent des souches bactériennes naturellement productrices de vitamine B12.

Bien entendu, il est toujours souhaitable, pour satisfaire aux besoins industriels, tant en alimentation animale qu'en pharmacie, de disposer de systèmes de production toujours plus performants.

Ainsi, les recherches actuelles portent d'abord sur l'amélioration de la productivité des souches naturellement productrices, en utilisant les techniques de la mutagénèse et de la recombinaison génétique.

La voie de la synthèse de la vitamine B12 est relativement complexe et maintenant bien connue.

Elle correspond, au niveau génétique, à trois régions impliquées dans la synthèse de différents précurseurs de la vitamine B12.

Plus précisément, les gènes impliqués dans la biosynthèse de la vitamine B12 par les bactéries semblent groupés en trois opérons : l'opéron cobI impliqué dans la biosynthèse de la cobinamide, l'opéron cobII impliqué dans la biosynthèse du dimethylbenzimidazole (DMB) et l'opéron cobIII codant pour une fonction permettant la réaction de la cobinamide et du DMB pour former la cobalamine. L'opéron cobI joue un rôle particulièrement important dans la mesure où une vingtaine de gènes sont impliqués dans la synthèse de la cobinamide.

On connaît plusieurs bactéries naturellement productrices, c'est-à-dire qui possèdent tous les gènes de la voie de biosynthèse. On peut citer de façon non limitative Bacillus megaterium, Propionobacterium shermanii, Pseudomonas putida et Agrobacterium tumefaciens.

En pratique, essentiellement les propionibactéries, c'est-à-dire les bactéries qui synthétisent de l'acide propionique telles que Propionobacterium freudenreichii et Propionobacterium shermanii, ainsi que Pseudomonas denitrificans, sont utilisées industriellement.

Chez ces bactéries naturellement productrices, certains auteurs ont déjà effectué le clonage et la propagation de certains groupes de gènes afin d'augmenter la quantité de gènes correspondant à des enzymes limitantes.

Ainsi Brey et al, Journal of Bacteriology Août 1986, pages 623-630 ont étudié des groupes de gènes de l'opéron cobI de Bacillus megaterium. Cameron et al, Journal of Bacteriology, Janvier 1989, pages 547-557 ont décrit le clonage de groupes de gènes de Pseudomonas denitrificans capables de complémenter, chez les bactéries Pseudomonas putida et Agrobacterium tumefaciens, des mutations cob. Un mutant cob est un mutant incapable de synthétiser la cobalamine (vitamine B12).

Toutefois, tous ces travaux ne visent qu'à améliorer la productivité de souches naturellement productrices de vitamine B12.

Or, il est également intéressant de pouvoir faire produire la vitamine B12 par d'autres bactéries qui ne la produisent pas naturellement.

Le déposant a maintenant trouvé qu'il est possible de faire produire de la vitamine B12 par la bactérie E.Coli en la transformant avec un ADN hétérologue comportant tout ou partie de la région cobI et permettant l'expression de ladite région dans E.Coli.

C'est pourquoi l'invention a pour objet une souche de E.Coli productrice de vitamine B12 transformée par un ADN hétérologue comportant tout ou partie de la région cobI et permettant l'expression de ladite région dans E.Coli.

La bactérie E.Coli a fait l'objet de nombreuses manipulations génétiques car elle présente une bonne aptitude à la transformation, et de nombreux systèmes de production dans E.Coli ont été éprouvés pour obtenir des produits d'intérêt industriel. Dans le cas de la vitamine B12, on peut donc penser que la découverte à la base de l'invention ouvre la possibilité de produire la vitamine B12 à l'échelle industrielle par mise en culture de E.Coli.

La région cobI hétérologue peut provenir de toute bactérie chez laquelle cette région a été identifiée. On peut citer, de façon non limitative, la région cobI de Citrobacter, de Salmonella typhimurium, et d'une manière générale de toute bactérie naturellement productrice de vitamine B12.

Dans la suite de la description, on s'intéressera plus particulièrement à la région cobI de Salmonella typhimurium.

L'ADN hétérologue utilisé pour transformer E.Coli comporte ou non des éléments assurant l'expression de la région cobI dans E.Coli. En effet, différentes possibilités sont envisageables. La région cobI peut utiliser des éléments propres à E.Coli, déjà présents dans la bactérie ou encore être introduite, portée par un vecteur, avec des éléments d'expression hétérologues. En tant que vecteur, on peut utiliser différents systèmes tels que les plasmides ou les cosmides.

Tout ou partie de la région cobI peut être introduite, dès lors que son expression conduit à la production de vitamine B12 par E.Coli.

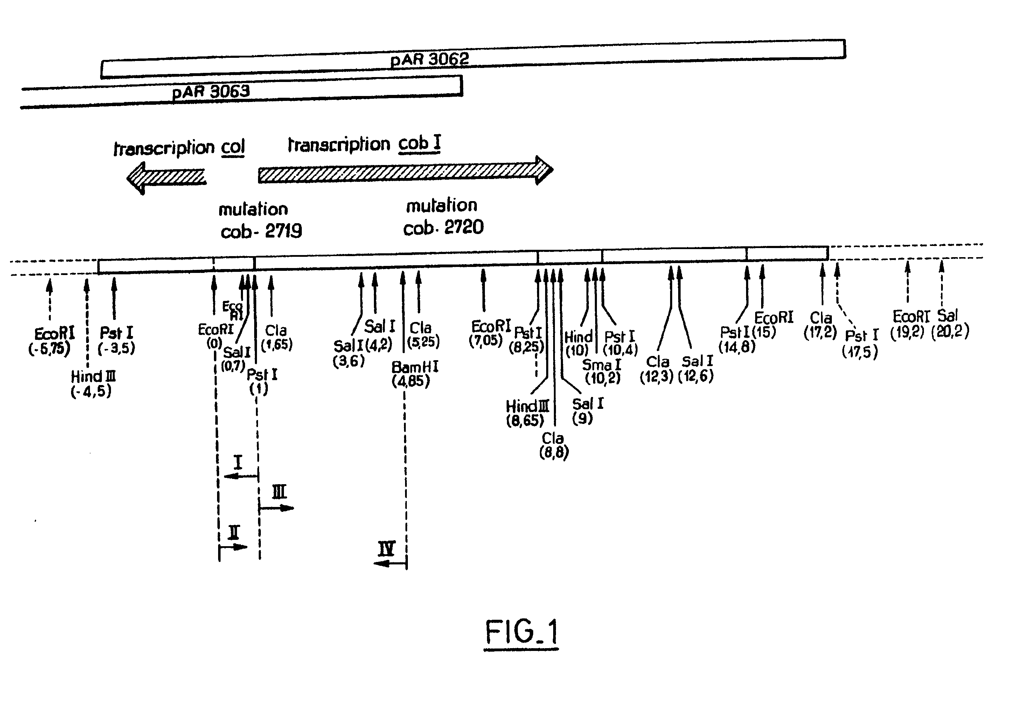

Ainsi, on a déterminé que la transcription cobI s'effectue à partir d'une portion de gène allant du site PstI(1) jusqu'au site PstI (8,25) tel qu'identifié à la figure 1 et on constate que le vecteur pAR3063 qui ne comprend pas les sites EcoRI (7,05) et PstI(1) (8,25) ne permet pas la production de vitamine B12, même s'il complémente cependant des mutations cob désignées par mutation cob-2719 et cob-2720. C'est pourquoi l'invention concerne plus particulièrement une souche de E.Coli transformée par un ADN hétérologue comportant la région cobI choisie parmi :

a) la région comprise entre les sites de restriction PstI(1) et PstI (8,25) telle que représentée sur le diagramme de la figure 1,

b) toute séquence d'ADN qui s'hybride à la région définie en a)

c) toute séquence d'ADN dégénérée en raison du code génétique par rapport aux séquences d'ADN définies en a) et b).

Plus particulièrement, la souche selon l'invention est telle que la région définie en a) présente, à partir de l'extrémité 5', la séquence suivante :

CCTGCAGCAC AGCCGCGATG GCCAAACAGC TTGGTTGCCC GGTCATCCTA
CTGGTGGACG GCAAGGCTGT TTCAACGTCG CTTGCGGCTA CCGTTATGGG
ATTTCAGCAT TCGACCCGAC CCTCAACCTC GCGGGCGTGA TTGTGAATCG
CGTCACTAGC CGACGCCCAC TATCACTCCT CAAAAATGCC ATTGAACATT
ATTGCTCACT GCCGGTACTG GGGTATGTCC CTCCTTGCGA CGGCGTCGAT
TACCTGCACG CCACTGGGGT TGATTACTGC CAGAGATCCT CGTCATCAGC
ATCATGGCAT GATTT

et à partir du site de restriction BamHI (4,85), elle présente, vers le site PstI(1), la séquence suivante :

GGATCCCTTT ATCCCGACGG CGGCAATCCA TGACAACAAC GCGCCAATAT
CCGCGCCCAG GCTAAATCGT TCGCCGCCAA ACGCCGGAAA CTGCGCCAAA
TGCCGTAAAC CGCCTGCCAG CGCATCGGCG TGGTCTATCG CTTCCAGCGC
CGCACGGTCA TCAAATGCCG TCCAGCGGGC CCCATTCCCA CGACCGTTAG
CATTGCAGCT CCTTAGCAAT CTCGTCGACG GGACGATTAC TGCCGAGAA

Suivant un mode de réalisation avantageux, la région cob I comprend également le site PstI (1) lui-même. Suivant un autre mode de réalisation, la région CobI, telle que définie en a) comprend également le site Pst (8,25) lui-même. Plus particulièrement, la région définie en a) comprend également la région comprise entre les sites de restriction PstI (1) et EcoRI (0) telle que représentée sur le diagramme de la figure 1, ayant, à partir de site PstI (1) vers le site EcoRI (0) la séquence suivante :

TAATTGGGGT CGACGCATAG CTGTCATACA ACCCCATGAC CCCTTCAATG

ACAGCGATAT CCGCCTGCCG CATTTGTTCG CAAAATAAGG CGTTGAGAAC

AGGAGGAGGA AGCATGAAAC TGTCAAGATT ACGGGACGCC ACGCCACAGA

TAGCGGTATG CCAGCCGGTA TCAAGGTAAT CTGGCCCAAC TTTAAACGGC

TGTACGCGCA

et à partir du site EcoRI(0) vers le site PstI(1) la séquence suivante :

CTCATCCTCC ATCGTTATGA ATAGCAATAT TTTGCTATTG CCGATTTTTG

ATAGTCTTTT GTATCTTAAT CATTTCAGAA AGAAAATTTA TXTGGTGTAA

CAATAAATTG TCATAGCGCA ACAAAATAAT AAAATTTAGG GCATAAACTG

CACCAGTTTT TATTTTTGTG ATGAAGTACA GTGTCAGGAA AGATAAGTTT

TATTACGCTT TGTCGATACG TTTTATCGTC AATATACCGG TAAGGATGAG

TAGATTAACG TCAGATGAGC AACCGCTCAG GCTATTGCTC ACAGAAATGT

AAACGTAGCA CATTATTAAT TGTCGTTATG GGTGTGCCGT ACAGCCATAA

CGTAACCACA GGTTGCCACA TTGTGGTAGG GAGGGGTGAA TCCCGCAGCC

CGCTGCTGTG ATGCTGAC

Enfin, l'invention couvre également les formes de réalisation dans lesquelles la région cobI définie en a), lorsqu'elle comprend la région telle que définie précédemment, comprend également les sites PstI (1) et EcoRI (0) eux-mêmes. Dans tous les modes de réalisation tels que définis précédemment, on peut prévoir que la région cobI définie en a) comporte également la région comprise entre les sites de restriction EcoRI (0) et PstI (-3,5), ainsi que le cas où ladite région cobI comporte toute la région allant du site PstI (-3,5) au site Cla (17,2), toujours selon le mode de représentation adopté dans le diagramme de la figure 1.

L'invention a également pour objet un procédé de préparation de vitamine B12 selon lequel on cultive une souche de E.Coli conforme à l'invention dans un milieu de culture approprié et on récupère la vitamine B12 obtenue.

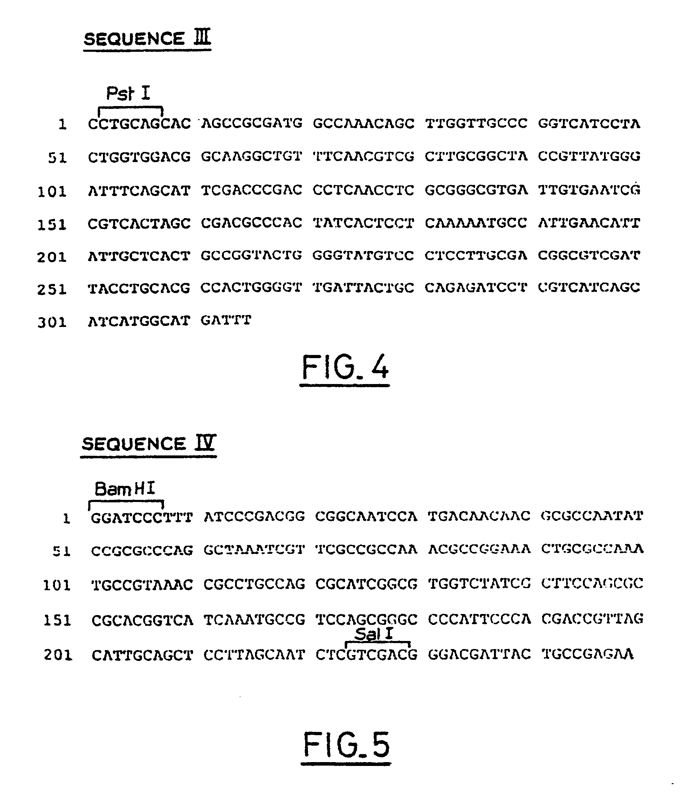

D'autres caractéristiques et avantages de l'invention apparaîtront au cours de la description détaillée suivante, accompagnée des figures 1 à 5, lesquelles représentent :

– Figure 1 : la carte de restriction d'une partie du génome de Salmonella typhimurium, avec en parallèle les fragments dudit génome contenus dans les plasmides pAR3062 et pAR3063. Les sites de restriction les plus courants sont indiqués par leur désignation suivie d'un chiffre entre parenthèses qui visualise pour la commodité de la figure, la position des sites par rapport au site EcoRI(0). Les flèches accompagnées de signes romains I, II, III, IV correspondent aux régions du génome dont la séquence a été déterminée. Les flèches épaisses situées au dessus de la carte de restriction symbolisent le sens de transcription de la région cobI d'une part et d'autre part du gène Col, qui correspond au gène Pdu mentionné dans le document Abstracts of the 90th Annual Meeting Am. Soc. for Microbiology 1990, 13-17 Mai, Anaheim, Californie. Les plasmides pAR3062 et pAR3063 sont représentés de façon à montrer leur zone de recouvrement avec les régions du génome de Salmonella typhimurium.

– Figure 2 : une partie de la séquence (séquence I) des plasmides pAR3062 et pAR3063 allant du site Pst(I) vers le site EcoRI(0) tels qu'indiqués à la figure 1.

– Figure 3 : une partie de la séquence (séquence II) des plasmides pAR3062 et pAR3063, allant du site EcoRI(0) vers le site PstI(1) tels qu'indiqués à la figure 1.

– Figure 4 : une partie de la séquence (séquence III) des plasmides pAR3062 et pAR3063 allant du site PstI(1) vers le site Bam HI (4,85) tels qu'indiqués à la figure 1.

– Figure 5 : une partie de la séquence (séquence IV) des plasmides pAR3062 et pAR3063, allant du site BamHI (4,85) vers le site PstI(1) tels qu'indiqués à la figure 1.

Les exemples de réalisation décrivent la préparation de deux vecteurs désignés respectivement par pAR3062 et pAR3063 dont l'un illustre l'invention et l'autre correspond à un essai comparatif, afin de mettre en évidence la structure minimale que doit avoir la portion de la région cobI pour résoudre le problème posé par l'invention.

Exemple : Préparation d'une souche de E.Coli productrice de vitamine B12.

Pour rechercher un ensemble de gènes apportant une fonction cobI active chez E.coli les expériences ont porté sur l'ADN de souches bactériennes dérivées de Salmonella typhimurium LT2. La souche AR2155 est un dérivé restriction négatif de la souche LT2 qui a servi de donatrice d'ADN.

Les bactéries ont été cultivées en milieu minimum (par litre : $Na_2HPO4$ 6 g, $KH_2PO4$ 3 g, NaCl 0,5 g, NH4Cl 1g, MgSO4 0,2 g, $CaCl_2$ 0,01 g, glucose 10 g, Casamino acides 1 g) puis récupérées par centrifugation et traitées en SDS 2 %, protéinase K 10 mg/ml pendant 3 heures à 40°C. L'ADN a ensuite été extrait par le phénol et précipité à l'éthanol en présence d'acétate de sodium 0,3 M. Les brins d'ADN ont été collectés puis remis sur un tampon Tris pH 7,5 50 mM, EDTA (1 mM) pour traitement à la RNase à 200 mcg/ml. L'ADN est extrait ensuite au chlorofome, précipité à l'éthanol, resuspendu et dialysé contre un tampon Tris pH 7,5 10mM, EDTA 10 mM, NaCl 10 mM.

Cet ADN chromosomique est ensuite digéré partiellement par l'enzyme de restriction Sau3A à des concentrations décroissantes allant de 2 unités par mcg d'ADN jusqu'à 0,015 unités par mcg d'ADN pendant 1 heure à 37°C. Les digestions sont arrêtées par l'ajout d'EDTA à la concentration finale de 20 mM et l'ADN ainsi digéré est placé sur un gel d'agarose à 0,4 % dans un champ d'environ 1 volt par cm jusqu'à migration complète du bleu de bromophénol utilisé comme marqueur de migration.

Les bandes de gel correspondant à une taille d'environ 25 kilobases sont découpées, mises dans un sac à dialyse dans un tampon Tris borate EDTA et placées dans une cuve d'électrophorèse. L'électroélution est effectuée à 200 volts pendant 1 heure avec inversion de courant pendant 2 minutes pour libérer l'ADN. L'ADN est filtré sur colonne de laine de verre puis extrait au phénol et au chloroforme. Il est ensuite précipité deux fois à l'éthanol et resuspendu sur un tampon Tris EDTA.

Cette préparation d'ADN est alors liguée à l'aide de la ligase dans le vecteur pLA2917 (L.N. Allen et R.S. Hanson, Journal of Bacteriology (1985) 161 pages 955-962). Ce vecteur est un cosmide portant, en plus d'un marqueur de résistance à la tétracycline, un marqueur de résistance à la kanamycine inactivé lors d'une coupure par l'enzyme BglII.

Ce vecteur pLA2917 est préalablement digéré complètement par l'enzyme BglII puis déphosphorylé à l'aide d'enzymes commerciales utilisées suivant les protocoles des fournisseurs.

Le produit de la ligation est ensuite encapsidé suivant la méthode de Hohn (Methods Enzymol. (1979) 68 pages 299-309) en utilisant des extraits d'encapsidation commerciaux (Stratagene cloning systems, La Jolla, California).

Ce ligat est ensuite amplifié dans la souche restriction négative de E.Coli, S17-1 (Simon R.,U. Priefer, et A. Pühler (1982) Biotechnology 1 pages 784-791) par infection de 5 ml de cette bactérie sensible (suspendue en MgSO4 0,01 M) avec 0,5 ml d'encapsidat, suivie d'un étalement sur 25 boîtes de milieu peptonée contenant de la tétracycline à 20 mcg/ml. Environ 12000 colonies sont obtenues et remises en suspension en MgSO4 0,01 M. Ce mélange sert à préparer un stock de bactériophage lambda virulent qui a pour titre $2.10^9$ pfu/ml.

La population de bactériophages ainsi obtenue contient des particules virales de lambda vir quine peuvent se développer chez Salmonella typhimurium (Harkki A. et Palva E.T. (1984) Mol. Gen. Genet. 195 pages 256-259) et des particules de cosmides qui peuvent s'y répliquer après infection, à condition d'utiliser une souche de Salmonella typhimurium sensible à l'adsorption du bactériophage lambda.

Pour réaliser la transformation, on utilise une souche de Salmonella typhimurium rendue sensible à l'infection par le bactériophage lambda (Palva E.T., Liljeström P., Harayama S. (1981) Mol. Gen. Genet. 181 pages 153-157) et porteuse de mutations restriction négatives, ci-après dénommée souche TN2540. Cette souche de Salmonella typhimurium comporte également une mutation cobI- (Jeter, R.M., B.M. Olivera et J. Roth (1984) J. Bacteriol. 170 pages 2078-2082) afin de rechercher des clones exprimant la région cobI.

Cette souche réceptrice, dénommée TN2540 cobI-, est infectée par la population de bactériophages obtenue précédemment à une multiplicité d'infection de 0,5 pour rechercher des clones devenus résistants à la tétracycline, puis parmi eux des clones devenus cobI+. De tels clones cobI+ sont trouvés à une fréquence d'environ 1 pour 500 clones résistants à la tétracycline examinés.

L'un des plasmides ainsi obtenu est dénommé pAR3062 et sa carte physique de restriction est déterminée à l'aide d'enzymes de restriction comme indiqué sur la figure 1. Le plasmide pAR3062 comprend toute la partie allant du site PstI (-3,5) au site PstI (17,5).

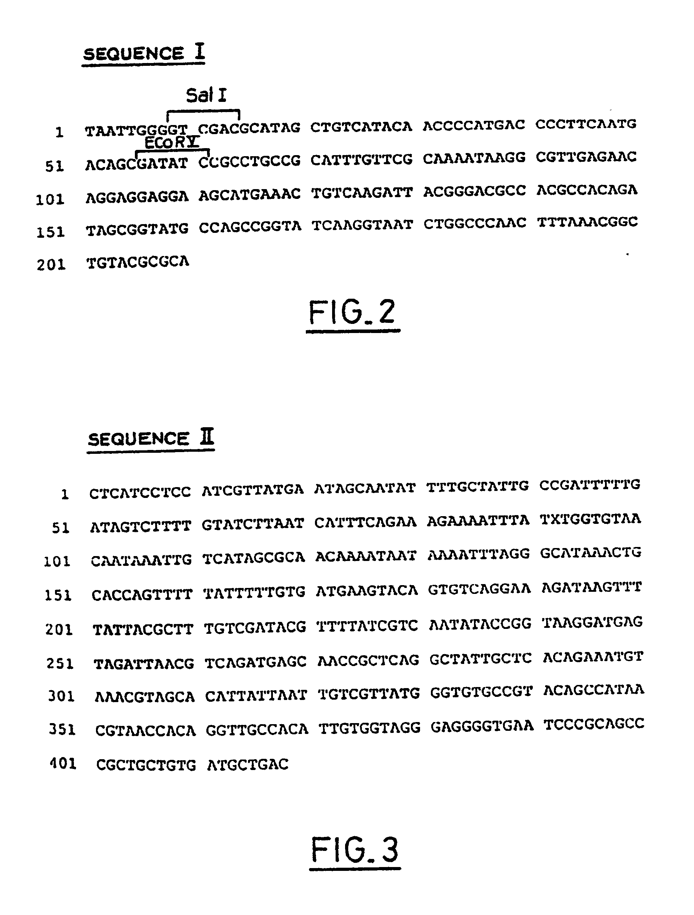

De plus, une portion de la région a été séquencée et les séquences I, II, III, IV correspondantes sont indiquées aux figures 2 à 5.

On prépare d'autre part un plasmide contrôle pAR3063 quine contient, du côté 3', que le site Cla (5,25), mais ne contient pas les sites EcoRI (7,05) et Pst I (8,25) tels qu'identifiés à la figure 1.

De même, une partie de ce plasmide a été séquencée et la partie séquencée se recouvre avec les séquences I, II, III, IV de pAR3062.

Le plasmide pAR3062 et le plasmide contrôle pAR3063 sont transférés dans la souche d'E.Coli K12 W3110. Ces deux souches ainsi que la souche de Salmonella Typhimurium AR2155 utilisée comme contrôle positif, sont cultivées sous anaérobie, en milieu liquide (par litre : extrait de levure 5 g, chlorure de cobalt 1 mg, dimethyl benzymidazole 50 mg pendant 48 heures à 37°C).

Les cultures sont ensuite traitées à 20 minutes à 20°C puis on extrait la vitamine B12 au phénol et des aliquots sont déposés sur une souche indicatrice E.Coli met $E^-$ sur milieu minimum sans méthionine. Les mutants met $E^-$ peuvent utiliser la vitamine B12 exogène pour contourner leur besoin en méthionine exogène. On détermine la quantité de vitamine B12 produite en mesurant les halos de croissance de la bactérie indicatrice.

La souche de Salmonella typhimurium AR2155 donne des halos de 22 mm (équivalent à 0, 10 mg/g de bactérie), la souche E.Coli W3110 (pAR3062) des halos de 20 mm (équivalent à 0,08 mg/g de bactérie) et la souche contrôle E.Coli W3110 (pAR3063) n'a pas donné de halo. Par conséquent, la bactérie E.Coli naturellement non productrice de vitamine B12 peut la produire lorsqu'elle est transformée conformément à l'invention.

Bien entendu, il est possible, sans sortir du cadre de l'invention de préparer des vecteurs d'expression dans E.Coli permettant de surexprimer la vitamine B12 grâce à la présence de promoteurs réputés forts dans E.Coli tels que les promoteurs PGK, Ptac, $P_L$, etc.

SEQ ID NO : 1

Type de séquence : Nucleotide
Longueur de la séquence : 210
Nombre de brins : simple
Configuration : linéaire
Type de molécule : ADN génomique
Origine Organisme : Salmonella
Propriétés : Séquence du système vitamine B12

Sal I

TAATTGGGGT CGACGCATAG CTGTCATACA ACCCCATGAC CCCTTCAATG 50

EcoRV

ACAGCGATAT CCGCCTGCCG CATTTGTTCG CAAAATAAGG CGTTGAGAAC 100

AGGAGGAGGA AGCATGAAAC TGTCAAGATT ACGGGACGCC ACGCCACAGA 150

TAGCGGTATG CCAGCCGGTA TCAAGGTAAT CTGGCCCAAC TTTAAACGGC 200

TGTACGCGCA 210

SEQ ID NO : 2

Type de séquence : Nucleotide
Longueur de la séquence : 418
Nombre de brins : simple
Configuration : linéaire
Type de molécule : ADN génomique
Origine Organisme : Salmonella
Propriétés : Séquence du système vitamine B12

CTCATCCTCC ATCGTTATGA ATAGCAATAT TTTGCTATTG CCGATTTTTG 50

ATAGTCTTTT GTATCTTAAT CATTTCAGAA AGAAAATTTA TXTGGTGTAA 100

CAATAAATTG TCATAGCGCA ACAAAATAAT AAAATTTAGG GCATAAACTG 150

CACCAGTTTT TATTTTTGTG ATGAAGTACA GTGTCAGGAA AGATAAGTTT 200

TATTACGCTT TGTCGATACG TTTTATCGTC AATATACCGG TAAGGATGAG 250

TAGATTAACG TCAGATGAGC AACCGCTCAG GCTATTGCTC ACAGAAATGT 300

AAACGTAGCA CATTATTAAT TGTCGTTATG GGTGTGCCGT ACAGCCATAA 350

CGTAACCACA GGTTGCCACA TTGTGGTAGG GAGGGGTGAA TCCCGCAGCC 400

CGCTGCTGTG ATGCTGAC 418

SEQ ID NO : 3

Type de séquence : Nucleotide
Longueur de la séquence : 315
Nombre de brins : simple
Configuration : linéaire
Type de molécule : ADN génomique
Origine Organisme : Salmonella
Propriétés : Séquence du système vitamine B12

Pst I

CCTGCAGCAC AGCCGCGATG GCCAAACAGC TTGGTTGCCC GGTCATCCTA 50

CTGGTGGACG GCAAGGCTGT TTCAACGTCG CTTGCGGCTA CCGTTATGGG 100

ATTTCAGCAT TCGACCCGAC CCTCAACCTC GCGGGCGTGA TTGTGAATCG 150

CGTCACTAGC CGACGCCCAC TATCACTCCT CAAAAATGCC ATTGAACATT 200

ATTGCTCACT GCCGGTACTG GGGTATGTCC CTCCTTGCGA CGGCGTCGAT 250

TACCTGCACG CCACTGGGGT TGATTACTGC CAGAGATCCT CGTCATCAGC 300

ATCATGGCAT GATTT 315

SEQ ID NO : 4

Type de séquence : Nucleotide
Longueur de la séquence : 249
Nombre de brins : simple
Configuration : linéaire
Type de molécule : ADN génomique
Origine Organisme : Salmonella
Propriétés : Séquence du système vitamine B12

BamHI

GGATCCCTTT ATCCCGACGG CGGCAATCCA TGACAACAAC GCGCCAATAT 50

CCGCGCCCAG GCTAAATCGT TCGCCGCCAA ACGCCGGAAA CTGCGCCAAA 100

TGCCGTAAAC CGCCTGCCAG CGCATCGGCG TGGTCTATCG CTTCCAGCGC 150

CGCACGGTCA TCAAATGCCG TCCAGCGGGC CCCATTCCCA CGACCGTTAG 200

Sal I

CATTGCAGCT CCTTAGCAAT CTCGTCGACG GGACGATTAC TGCCGAGAA 249

## Revendications

1. Souche de E.Coli productrice de vitamine B12 transformée par un ADN hétérologue comportant tout ou partie de la région cobI et permettant l'expression de ladite région dans E.Coli.

2. Souche selon la revendication 1, caractérisée en ce qu'il s'agit de la région cobI de Salmonella typhimurium.

3. Souche selon la revendication 2, caractérisée en ce qu'il s'agit de la région cobI choisie parmi :
   a) la région comprise entre les sites de restriction PstI(1) et PstI (8,25) telle que représentée sur le diagramme de la figure 1,
   b) toute séquence d'ADN qui s'hybride à la région définie en a)
   c) toute séquence d'ADN dégénérée en raison du code génétique par rapport aux séquences d'ADN définies en a) et b).

4. Souche selon la revendication 3, caractérisée en ce que, la région définie en a) présente, à partir de l'extrémité 5' la séquence suivante :

```
CCTGCAGCAC AGCCGCGATG GCCAAACAGC TTGGTTGCCC GGTCATCCTA
CTGGTGGACG GCAAGGCTGT TTCAACGTCG CTTGCGGCTA CCGTTATGGG
ATTTCAGCAT TCGACCCGAC CCTCAACCTC GCGGGCGTGA TTGTGAATCG
CGTCACTAGC CGACGCCCAC TATCACTCCT CAAAAATGCC ATTGAACATT
ATTGCTCACT GCCGGTACTG GGGTATGTCC CTCCTTGCGA CGGCGTCGAT
TACCTGCACG CCACTGGGGT TGATTACTGC CAGAGATCCT CGTCATCAGC
ATCATGGCAT GATTT
```

et à partir du site de restriction BamHI (4,85), elle présente, vers le site PstI(1), la séquence suivante :

```
GGATCCCTTT ATCCCGACGG CGGCAATCCA TGACAACAAC GCGCCAATAT
CCGCGCCCAG GCTAAATCGT TCGCCGCCAA ACGCCGGAAA CTGCGCCAAA
TGCCGTAAAC CGCCTGCCAG CGCATCGGCG TGGTCTATCG CTTCCAGCGC
CGCACGGTCA TCAAATGCCG TCCAGCGGGC CCCATTCCCA CGACCGTTAG
CATTGCAGCT CCTTAGCAAT CTCGTCGACG GGACGATTAC TGCCGAGAA
```

5. Souche selon la revendication 3, caractérisée en ce que ladite région cobI comprend également le site PstI (1) lui-même.

6. Souche selon l'une des revendications 3 à 5, caractérisée en ce que ladite région cobI, définie en a) comprend également le site Pst (8,25) lui-même.

7. Souche selon l'une des revendications 3 à 6, caractérisée en ce que la région définie en a) comprend également la région comprise entre les sites de restriction PstI (1) et EcoRI(0) telle que représentée sur le diagramme de la figure 1, ayant, à partir de site PstI(1) vers le site EcoRI(0) la séquence suivante :

TAATTGGGGT CGACGCATAG CTGTCATACA ACCCCATGAC CCCTTCAATG
ACAGCGATAT CCGCCTGCCG CATTTGTTCG CAAAATAAGG CGTTGAGAAC
AGGAGGAGGA AGCATGAAAC TGTCAAGATT ACGGGACGCC ACGCCACAGA
TAGCGGTATG CCAGCCGGTA TCAAGGTAAT CTGGCCCAAC TTTAAACGGC
TGTACGCGCA

et à partir du site EcoRI(0) vers le site PstI(1) la séquence suivante :

CTCATCCTCC ATCGTTATGA ATAGCAATAT TTTGCTATTG CCGATTTTTG
ATAGTCTTTT GTATCTTAAT CATTTCAGAA AGAAAATTTA TXTGGTGTAA
CAATAAATTG TCATAGCGCA ACAAAATAAT AAAATTTAGG GCATAAACTG
CACCAGTTTT TATTTTTGTG ATGAAGTACA GTGTCAGGAA AGATAAGTTT
TATTACGCTT TGTCGATACG TTTTATCGTC AATATACCGG TAAGGATGAG
TAGATTAACG TCAGATGAGC AACCGCTCAG GCTATTGCTC ACAGAAATGT
AAACGTAGCA CATTATTAAT TGTCGTTATG GGTGTGCCGT ACAGCCATAA
CGTAACCACA GGTTGCCACA TTGTGGTAGG GAGGGGTGAA TCCCGCAGCC
CGCTGCTGTG ATGCTGAC

**8.** Souche selon la revendication 7, caractérisée en ce que la région cobI définie en a) comprend également les sites PstI(1) et EcoRI(0) eux-mêmes.

**9.** Souche selon l'une des revendications 3 à 8, caractérisée en ce que la région cobI définie en a) comporte également la région comprise entre les sites de restriction EcoRI(0) et PstI(-3,5) telle que représentée sur le diagramme de la figure 1.

**10.** Souche selon l'une des revendications 3 à 9, caractérisée en ce que la région cobI définie en a) comporte toute la région allant site PstI (-3,5) au site Cla (17,2) selon les indications données dans le diagramme de la figure 1.

**11.** Procédé de préparation de vitamine B 12 caractérisé en ce qu'on cultive une souche de <u>E.Coli</u> selon l'une des revendications précédentes, dans un milieu de culture approprié et on récupère la vitamine B12 obtenue.

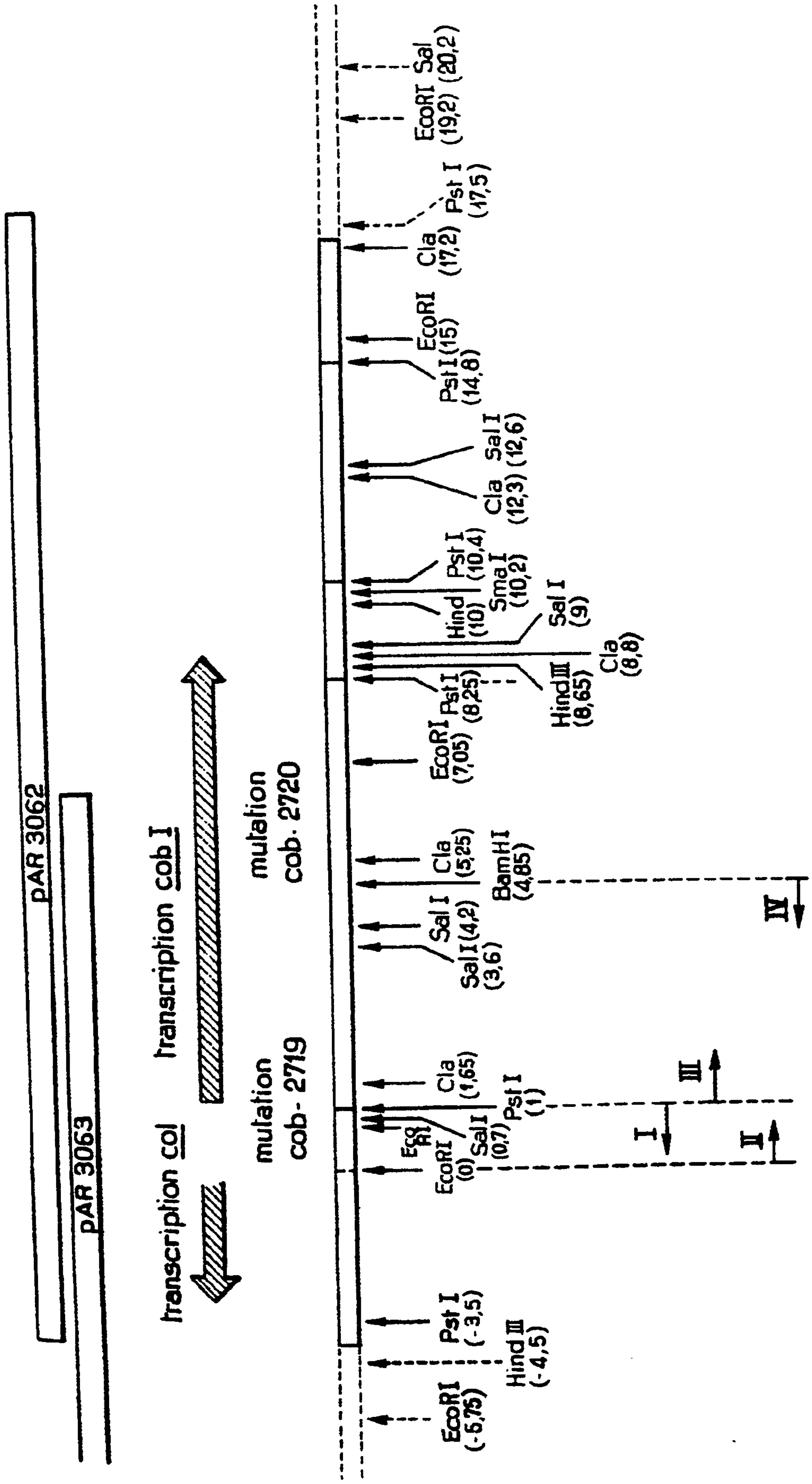
pAR 3062
pAR 3063
transcription col
transcription cob I
mutation cob-2719
mutation cob-2720
EcoRI (-5,75)
Hind III (-4,5)
Pst I (-3,5)
EcoRI (0)
Eco RI
Sal I (0,7)
Pst I (1)
Cla (1,65)
Sal I (3,6)
Sal I (4,2)
BamHI (4,85)
Cla (5,25)
EcoRI (7,05)
Pst I (8,25)
Hind III (8,65)
Cla (8,8)
Sal I (9)
Hind (10)
Sma I (10,2)
Pst I (10,4)
Cla (12,3)
Sal I (12,6)
Pst I (14,8)
EcoRI (15)
Cla (17,2)
Pst I (17,5)
EcoRI (19,2)
Sal (20,2)
I
II
III
IV

FIG_1

**SEQUENCE I**

Sal I

```
  1  TAATTGGGGT CGACGCATAG CTGTCATACA ACCCCATGAC CCCTTCAATG
                ECoRV
 51  ACAGCGATAT CCGCCTGCCG CATTTGTTCG CAAAATAAGG CGTTGAGAAC
101  AGGAGGAGGA AGCATGAAAC TGTCAAGATT ACGGGACGCC ACGCCACAGA
151  TAGCGGTATG CCAGCCGGTA TCAAGGTAAT CTGGCCCAAC TTTAAACGGC
201  TGTACGCGCA
```

FIG. 2

**SEQUENCE II**

```
  1  CTCATCCTCC ATCGTTATGA ATAGCAATAT TTTGCTATTG CCGATTTTTG
 51  ATAGTCTTTT GTATCTTAAT CATTTCAGAA AGAAAATTTA TXTGGTGTAA
101  CAATAAATTG TCATAGCGCA ACAAAATAAT AAAATTTAGG GCATAAACTG
151  CACCAGTTTT TATTTTTGTG ATGAAGTACA GTGTCAGGAA AGATAAGTTT
201  TATTACGCTT TGTCGATACG TTTTATCGTC AATATACCGG TAAGGATGAG
251  TAGATTAACG TCAGATGAGC AACCGCTCAG GCTATTGCTC ACAGAAATGT
301  AAACGTAGCA CATTATTAAT TGTCGTTATG GGTGTGCCGT ACAGCCATAA
351  CGTAACCACA GGTTGCCACA TTGTGGTAGG GAGGGGTGAA TCCCGCAGCC
401  CGCTGCTGTG ATGCTGAC
```

FIG. 3

## SEQUENCE III

Pst I

1 CCTGCAGCAC AGCCGCGATG GCCAAACAGC TTGGTTGCCC GGTCATCCTA

51 CTGGTGGACG GCAAGGCTGT TTCAACGTCG CTTGCGGCTA CCGTTATGGG

101 ATTTCAGCAT TCGACCCGAC CCTCAACCTC GCGGGCGTGA TTGTGAATCG

151 CGTCACTAGC CGACGCCCAC TATCACTCCT CAAAAATGCC ATTGAACATT

201 ATTGCTCACT GCCGGTACTG GGGTATGTCC CTCCTTGCGA CGGCGTCGAT

251 TACCTGCACG CCACTGGGGT TGATTACTGC CAGAGATCCT CGTCATCAGC

301 ATCATGGCAT GATTT

FIG. 4

## SEQUENCE IV

BamHI

1 GGATCCCTTT ATCCCGACGG CGGCAATCCA TGACAACAAC GCGCCAATAT

51 CCGCGCCCAG GCTAAATCGT TCGCCGCCAA ACGCCGGAAA CTGCGCCAAA

101 TGCCGTAAAC CGCCTGCCAG CGCATCGGCG TGGTCTATCG CTTCCAGCGC

151 CGCACGGTCA TCAAATGCCG TCCAGCGGGC CCCATTCCCA CGACCGTTAG

Sal I

201 CATTGCAGCT CCTTAGCAAT CTCGTCGACG GGACGATTAC TGCCGAGAA

FIG. 5

Office européen des brevets

# RAPPORT DE RECHERCHE EUROPEENNE

Numero de la demande

EP 91 40 1643

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | JOURNAL OF BACTERIOLOGY, vol. 169, no. 7, juillet 1987, pages 3189-3198, American Society for Microbiology; R.M. JETER et al.: "Cobalamin (Vitamin B12) biosynthetic genes of Salmonella typhimurium"<br>* Le document en entier *<br>--- | 1-11 | C 12 N 15/52<br>C 12 P 19/42<br>C 12 N 1/21 //<br>(C 12 N 15/52<br>C 12 R 1:42 )<br>(C 12 N 1/21<br>C 12 R 1:19 ) |
| D,A | JOURNAL OF BACTERIOLOGY, vol. 167, no. 2, août 1986, pages 623-630, American Society for Microbiology; R.N. BREY et al.: "Cloning of multiple genes involved with cobalamin (Vitamin B12) biosynthesis in Bacillus megaterium"<br>* Le document en entier *<br>----- | 1 | |

| DOMAINES TECHNIQUES RECHERCHES (Int. Cl.5) |
|---|
| C 12 N<br>C 12 P<br>C 12 R |

Le présent rapport a été établi pour toutes les revendications

| Lieu de la recherche | Date d'achèvement de la recherche | Examinateur |
|---|---|---|
| LA HAYE | 11-09-1991 | ANDRES S.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)